Europäisches Patentamt

European Patent Office

Office européen des brevets

(11) Veröffentlichungsnummer: **0 373 332**
**A1**

## (12) EUROPÄISCHE PATENTANMELDUNG

(21) Anmeldenummer: **89119971.3**

(22) Anmeldetag: **27.10.89**

(51) Int. Cl.⁵: **G01N 3/36, G01N 21/88, G01N 29/04, A61B 5/103**

(30) Priorität: **06.12.88 DE 3840998**

(43) Veröffentlichungstag der Anmeldung:
**20.06.90 Patentblatt 90/25**

(84) Benannte Vertragsstaaten:
**DE FR GB SE**

(71) Anmelder: **BATTELLE-INSTITUT E.V.**
**Am Römerhof 35 Postfach 90 01 60**
**D-6000 Frankfurt am Main 90(DE)**

(72) Erfinder: **Weiss, Rainer**
**Stettiner Strasse 48**
**D-6234 Hattersheim(DE)**

(54) **Vorrichtung zum zerstörungsfreien Aufspüren von Inhomogenitäten in elastischen Stoffen.**

(57) Vorrichtung zum zerstörungsfreien Aufspüren von Inhomogenitäten in elastischen Stoffen mit einer Belastungseinrichtung (18) zur Verformung des Stoffs (14), die gepulste Druckwellen auf eine mittels einer Beleuchtungseinrichtung (10) beleuchteten Stelle des Stoffs aussendet, wobei die Verformung der Oberfläche des Stoffs mittels einer optischen, berühungslos arbeitenden Abstands-Meßvorrichtung ermittelt wird, dadurch gekennzeichnet, daß die Belastungseinrichtung (18) als Flüssigkeitsstrahlgerät ausgebildet ist.

Die Erfindung kann durch eine berührungslos arbeitende Abstands-Meßvorrichtung ergänzt werden, die weitere Aufschlüsse über die Art der gemessenen Inhomogenitäten gibt.

## Vorrichtung zum zerstörungsfreien Aufspüren von Inhomogenitäten in elastischen Stoffen.

Die Erfindung betrifft eine Vorrichtung zum zerstörungsfreien Aufspüren von Inhomogenitäten in elastischen Stoffen, vorzugsweise in elastischen Werkstoffen, mit einer Belastungseinrichtung zur Verformung des Stoffs, die gepulste Druckwellen auf eine mittels einer Beleuchtungseinrichtung beleuchtete Stelle des Stoffs aussendet, wobei die Verformung der Oberfläche des Stoffs mittels einer optischen, berührungslos arbeitenden Abstandsmeßvorrichtung ermittelt wird.

Eine derartige Vorrichtung, mit der die Elastizität der Haut untersucht werden soll, ist in der deutschen Offenlegungsschrift 36 12 312 beschrieben. Dort sendet die Belastungseinrichtung gepulste Druckluftwellen auf die zu untersuchende Oberfläche der Haut.

Will man verhältnismäßig weiche Stoffe mit dieser Vorrichtung untersuchen, so ist das dort beschriebene Verfahren grundsätzlich anwendbar. Es versagt aber, wenn härtere Stoffe mit dieser Vorrichtung untersucht werden sollen, und zwar weil die gepulste Druckluft hierzu nicht ausreichend Energie hat. So sind dort die Druckschwankungen auf einen verhältnismäßig geringen Bereich zwischen 0 und 1 bar beschränkt. Außerdem muß dort der Druckgeber über eine Führungshülse in einem fest vorgegebenen Abstand für die Meßdauer auf die zu prüfende Überfläche aufgesetzt werden. Dadurch wird die Handhabbarkeit dieser bekannten Vorrichtung erschwert.

Demgegenüber liegt der Erfindung die Aufgabe zugrunde, eine Vorrichtung mit den eingangs genannten Merkmalen vorzuschlagen, die derart ausgebildet ist, daß eine kontinuierliche Abtastung großer Bereiche des zu prüfenden Stoffs bei geringer Störempfindlichkeit und sofortiger Fehleranzeige gegeben ist, wobei die für die Prüfung notwendigen höheren Arbeitsdrücke zur Detektion auch tiefer liegender Inhomogenitäten aufgebracht werden können.

Zur Lösung dieser Aufgabe ist die Erfindung dadurch gekennzeichnet, daß die Belastungseinrichtung als Flüssigkeitsstrahlgerät ausgebildet ist.

Durch die Verwendung von mindetens einer Druckwelle, die aus einem gepulsten Flüssigkeitsstrahl besteht, lassen sich auf einfache Weise Störungen bzw. Inhomogenitäten in Werkstoffen ermitteln, wobei dann die druckbelastete Stelle mittels der Lichtquelle beleuchtet wird und an der Meßstelle ein Lichtpunkt erzeugt wird. Die Lageveränderung des Lichtpunktes infolge der Oberflächenverformung kann dann durch den positionsempfindlichen Detektor nach dem Triangulationsverfahren (siehe Prospekt "Keyence Corp.", Cat. No. MLC-KJ-01) ermittelt werden. Hierzu ist es vorteilhaft, daß die Beleuchtungseinrichtung aus einem Lasergerät besteht, wobei der an der belasteten Stelle gestreute Laserstrahl mit einer Linse auf den positionsempfindlichen Detektor abgebildet wird. Dieser ist mit einer Auswerteeinheit verbunden. Als Bezugsgröße dient die Messung am gleichen Meßort ohne Druckbelastung. Hierdurch lassen sich auf einfache Weise von der Norm abweichende Verformungen des Werkstoffes ermitteln und über die Auswerteeinheit bestimmen.

Im folgenden wird die Erfindung anhand eines Ausführungsbeispieles näher erläutert. Es zeigt:

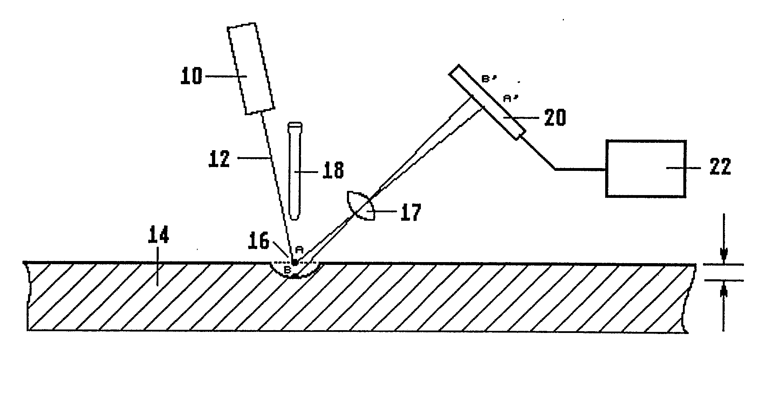

Fig. 1 - eine Vorrichtung zum zerstörungsfreien Aufspüren von Inhomogenitäten mit einem Lasergerät 10 und einer Belastungseinrichtung 18.

Das Lasergerät 10 sendet einen Lichtstrahl 12 auf ein Werkstück 14, der einen Lichtpunkt 16 auf dem Werkstoff 14 erzeugt. Oberhalb des Lichtpunktes 16 befindet sich eine Belastungseinrichtung 18, die auch als Flüssigkeitsstrahlgerät ausgebildet ist und die auf dem Werkstück 14 im Bereich des Lichtpunktes 16 eine zerstörungsfreie Verformung hervorruft. Der Lichtpunkt 16 wird über eine Linse 17 auf einen positionsempfindlichen Detektor 20 abgebildet. Mit Hilfe des Detektors 20 wird die Verformung des Werktückes ermittelt. Als Bezugsgröße dient die Messung am gleichen Meßort ohne Druckbelastung (Abstand zwischen A und B). Abweichende Verformungen erlauben somit Rückschlüsse auf etwaige Einschlüsse bzw. Inhomogenitäten des Werktoffes. Derartige Messungen werden fortlaufend bzw. kontinuierlich wiederholt, so daß über die gesamte Fläche des Werkstoffes ein Zustandsdiagramm mittels einer Auswerteeinheit 22 angefertigt und entsprechend ausgewertet wird, um somit Fehler über die Gesamtfläche eines Werkstückes direkt anzuzeigen. Die Auswerteeinheit 22 verarbeitet die unterschiedlichen Meßgrößen aus der Verformung der Werkstoffoberfläche.

Ferner ist es möglich, über eine zusätzliche Einrichtung die betreffenden Meßstellen farblich zu markieren, um nach Abschluß des Meßverfahrens die Inhomogenitäten im Werkstoff lokalisieren zu können.

Das Gerät kann auch in einem mit einer geeigneten Flüssigkeit gefüllter Behälter, z.B.in einer Wanne, arbeiten. Dadurch erhält man besonders gute Ankopplungsbedingungen. Dies empfiehlt sich insbesondere bei medizinischen Anwendungen, bei denen dann beispielsweise Muskelverhärtungen, Blutergüsse und dergleichen aufgesprüt und untersucht werden.

Die Flüssigkeit kann hierbei den besonderen Bedingungen angepaßt werden.

Auch andere Stoffe (Werkstoffe) lassen sich mit dem Gerät untersuchen, und zwar in Luft oder in einer geeigneten Flüssigkeit.

Wenn das Gerät 10 als Ultraschall-Impulssender ausgebildet ist, hat man eine berührungslos arbeitende Abstandsmeßvorrichtung, die die Tiefe A-B der elastischen Verformung mißt. Dies ermöglicht Rückschlüsse auf die Art und Stärke der betreffenden Imhomogenität.

Die von der Meßstelle 16 reflektierten Impulse werden hierbei von einem in das Gerät 10 eingebauten Empfänger empfangen und die Signale werden nach dem Laufzeit-Meßverfahren ausgewertet oder die reflektierten Signale werden von dem Empfänger 20 mit der Auswerteeinheit 22 ausgewertet.

Auch diese Messungen könnten mit einer mit Druckluft arbeitenden Belastungseinrichtung, wie nach dem eingangs geschilderten Stand der Technik, nicht durchgeführt werden.

**Ansprüche**

1. Vorrichtung zum zerstörungsfreien Aufspüren von Inhomogenitäten in elastischen Stoffen mit einer Belastungseinrichtung (18) zur Verformung des Stoffs (14), die gepulste Druckwellen auf eine mittels einer Beleuchtungseinrichtung (10) beleuchteten Stelle des Stoffs aussendet, wobei die Verformung der Oberfläche des Stoffs mittels einer optischen, berührungslos arbeitenden Abstandsmeßvorrichtung ermittelt wird,
**dadurch gekennzeichnet,**
daß die Belastungseinrichtung (18) als Flüssigkeitsstrahlgerät ausgebildet ist.

2. Vorrichtung nach Anspruch 1,
**dadurch gekennzeichnet,**
daß die Beleuchtungseinrichtung ein Lasergerät (10) aufweist, wobei der an der beleuchteten Stelle entstehende Lichtpunkt von der Abstandsmeßvorrichtung erfaßt wird, die aus einer Linse (17) und einem positionsempfindlichen Detektor (20) besteht, auf dem die Lichtpunkte abgebildet werden und an dem eine Auswerteeinheit angeschlossen ist.

3. Vorrichtung nach Anspruch 1 oder 2,
**dadurch gekennzeichnet,**
daß ein positionsempfindlicher Detektor (20) als berührungsloses optisches Meßgerät nach dem Triangulationsverfahren aufgebaut ist.

4. Vorrichtung nach Anspruch 1 oder 2,
**dadurch gekennzeichnet,**
daß ein positionsempfindlicher Detektor (20) als koaxialer Sensor in Form eines chromatischen Sensors aufgebaut ist.

5. Vorrichtung nach Anspruch 1 oder 2,
**dadurch gekennzeichnet,**
daß der positionsempfindliche Detektor (20) als koaxialer Sensor in Form eines Fokusdetektors aufgebaut ist.

6. Vorrichtung nach Anspruch 1 oder 2,
**dadurch gekennzeichnet,**
daß der positionsempfindliche Detektor (20) als koaxialer Sensor in Form eines Schärfentiefedetektors aufgebaut ist.

7. Vorrichtung nach einem der Ansprüche 1 bis 6,
**dadurch gekennzeichnet,**
daß sie eine Einrichtung zur farblichen Markierung der aufgefundenen Inhomogenitäten aufweist.

8. Vorrichtung nach einem der Ansprüche 1 bis 7,
**dadurch gekennzeichnet,**
daß die Übertragung der Flüssigkeitsstrahlen von der Belastungseinrichtung (18) auf die zu prüfende Stelle (16) über eine Flüssigkeit erfolgt.

9. Vorrichtung zum zerstörungsfreien Aufspüren von Inhomogenitäten in elastischen Stoffen mit einer Belastungseinrichtung (18) zur Verformung des Stoffs (14), die gepulste Druckwellen auf eine Stelle des Stoffs aussendet, wobei die Verformung der Oberfläche des Stoffs mittels einer berühungslos arbeitenden Abstandsmeßvorrichtung ermittelt wird,
**dadurch gekennzeichnet,**
daß die Belastungseinrichtung (18) als Flüssigkeitsstrahlgerät ausgebildet ist und daß eine Ultraschall-Abstandsmeßvorrichtung (10,12,20,22) vorgesehen ist, die die lokale Verformung mißt.

10. Vorrichtung nach Anspruch 9,
**dadurch gekennzeichnet,**
daß die Übertragung der Flüssigkeitsstrahlen von der Belastungseinrichtung (18) auf die zu prüfende Stelle (16) über eine Flüssigkeit erfolgt.

EP 0 373 332 A1

10

12 18

14 16

17

B' A' 20

22

A
B

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | ADVANCES IN INSTRUMENTATION Band 43, Nr. 2, 1988, Seiten 863-874, Research Triangle Park, NC, USA; P. CIELO et al.: "Optical inspection of sheet material for pinholes or defects by speckle-averaging techniques" * Seiten 863-865; Figuren 1,6 * | 1,2,7 | G 01 N 3/36 G 01 N 21/88 G 01 N 29/04 A 61 B 5/103 |
| A | idem --- | 3-6 | |
| D,Y | DE-A-3 612 312 (L'OREAL) * Zusammenfassung; Figur 2 * --- | 1,2,7 | |
| A | GB-A-1 106 485 (BRITISH HOVERCRAFT CORP. LTD.) * Seite 2, Zeilen 10-21 * --- | 1,9 | |
| Y | GB-A-2 138 138 (UNITED KINGDOM ATOMIC ENERGY AUTHORITY) * Zusammenfassung; Seite 1, Zeilen 71-87; Figur 1 * | 9 | |
| A | --- | 10 | |
| Y | DE-A-3 304 503 (SCHWARZHAUPT MEDIZINTECHNIK GMBH) * Zusammenfassung; Seite 5, Zeile 10 - Seite 7, Zeile 25; Figur 1 * --- | 9 | RECHERCHIERTE SACHGEBIETE (Int. Cl.5) G 01 N G 01 B G 01 L A 61 B |
| A | MATERIALS EVALUATION Band 34, Mai 1976, Seiten 45A,46A, Evanston, Illinois, USA; R.K. ERF: "HNDT Is Alive and Well" * Figur 2 * --- | 1-6 | |
| A | US-A-4 381 663 (W.M. SWANSON) * Zusammenfassung * ----- | 1,8-10 | |

Der vorliegende Recherchenbericht wurde für alle Patentansprüche erstellt

| Recherchenort | Abschlußdatum der Recherche | Prüfer |
|---|---|---|
| BERLIN | 22-02-1990 | JOHNSON K M |